Europäisches Patentamt

European Patent Office

Office européen des brevets

(11) Publication number: **0 223 978 B1**

# EUROPEAN PATENT SPECIFICATION

(45) Date of publication of patent specification: **19.08.92**  (51) Int. Cl.5: **G01N 33/80**

(21) Application number: **86113653.9**

(22) Date of filing: **03.10.86**

The file contains technical information submitted
after the application was filed and not included in
this specification

(54) **Diagnostic test.**

(30) Priority: **11.10.85 US 786617**

(43) Date of publication of application:
**03.06.87 Bulletin 87/23**

(45) Publication of the grant of the patent:
**19.08.92 Bulletin 92/34**

(84) Designated Contracting States:
**AT BE CH DE FR GB IT LI NL**

(56) References cited:
**EP-A- 63 810**      **EP-A- 117 019**
**EP-A- 217 845**     **WO-A-83/03477**
**WO-A-85/01354**     **US-A- 3 074 853**
**US-A- 3 502 437**    **US-A- 4 275 053**

(73) Proprietor: **ABBOTT LABORATORIES**
**14th Street and Sheridan Road North St**
**North Chicago, Illinois 60064(US)**

(72) Inventor: **Nevalainen, David E.**
**6455 W. English Meadow, Apt. F208**
**Greenfield, WI 53220(US)**

(74) Representative: **Modiano, Guido et al**
**MODIANO, JOSIF, PISANTY & STAUB**
**Modiano & Associati Via Meravigli, 16**
**I-20123 Milano(IT)**

**Description**

The present invention relates generally to methods for the immunological detection of antigenic determinants and specifically to methods and devices for determining blood group classifications from blood or serum samples.

Red blood cells are characterized by the presence of a variety of immunogenic markers which are of relevance to blood transfusions as well as to organ transplantation. A variety of blood group systems each comprising a number of related antigens is known. The major systems include the ABO, D (Rh), Lewis, I, Kell, P, MN, Lutheran, Kidd and Duffy systems. These systems and others are discussed in P.D. Issitt and C.H. Issitt, Applied Blood Group Serology, pp. 353-360 (Spectra Biologicals, 1975). The chemical determinants of the blood group antigens are of either polysaccharide or protein composition. Polysaccharide antigens occur as either glycoproteins or glycolipids and the determining structure usually consists of more than one sugar moeity. The blood group antigens are present at birth and remain intact throughout the life of an individual.

The blood serum of most individuals contains antibodies to ABO blood group antigens. The serum of an individual will not contain antibodies reactive with the antigens characteristic of that individual's blood cells but will often contain antibodies reactive with blood group antigens not characteristic of that individual's red blood cells (blood type). Such antibodies are not present at birth but gradually develop through life as a result of sensitization occurring as a consequence of transfusion or pregnancy as well as through exposure to non-blood antigens which abound in nature.

The importance of blood group serology was first recognized during attempts to transfuse blood from on individual to another. Early recipients of blood transfusions frequently became sick and occasionally died. It was discovered that antibodies present in the serum of transfusion recipients often agglutinated donated blood cells when the blood groups of the donor and recipient were different. Out of an early set of experiments the ABO major blood type system was described. The ABO blood group includes types O, A, B and AB as well as various subtypes of type A and type B. Individuals with O type blood were originally believed to have red blood cells with no specific blood group antigens on their surface. It is now known that O group blood cells display an antigen known as the H antigen. Individuals with O type blood have anti-A and anti-B antibodies in their serum. Individuals with O type blood have red blood cells with A antigen on their surface and have anti-B antibodies in their serum. Individuals with type B blood have B antigen on the surface of their red blood cells and have anti-A antibodies in their serum. Those with type AB blood have both A and B antigen on the surface of their red blood cells and have no anti-A or anti-B antibodies circulating in their serum.

The most important blood group system other than the ABO system is the D (Rh) system. The system is often described in conjunction with the ABO system with the type suffix "positive" or "negative" as in "O-positive" or "B-negative" to indicate the presence or absence of the D (Rh) antigen. The Rh designation is something of a misnomer and was originally used because an antigen similar to the D antigen was detected by serum produced in rabbits immunized with Rhesus monkey red blood cells. The "Rh antigen" is actually a family of antigens which vary considerably in their capacity to immunize. The D antigen is the most potent and is the antigen commonly referred to when speaking of the Rh-positive or Rh-negative types. It is also this antigen which is most commonly involved in sensitization by transfusion or pregnancy. Other antigens of the D (Rh) system do exist but these are less potent and, consequently, cause less difficulty in transfusion or fetomaternal incompatability.

In some patients, the expression of the D antigen, although positive, appears to be particularly weak. This is often due to the presence of a varient form of the D antigen known as $D^{\mu}$. The varient is of interest because its presence may be missed without special care. It may be capable of stimulating antibody production in D-negative recipients.

In addition to the antigenic determinants which define the ABO and D (Rh) blood groups, there exist numerous other antigens which are presented on the surface of red blood cells and which define blood groups. In some cases the antigens are extremely rare, while in others they are found in an overwhelming majority of the population. In many cases, however, severe reactions can occur as with hemolytic disease of the newborn. The Kell blood group system (K antigen) is occasionally involved in cross-matching difficulties and hemolytic disease of the newborn. The Kidd (JK) antigen system is also occasionally responsible for delayed transfusion reactions. These major blood groups are also of interest for various anthropological studies as well as in parental exclusion testing and criminal forensics work.

Conventional liquid phase methods for blood group determinations generally comprise agglutination tests and cell lysis tests. In agglutination tests, samples of blood are mixed with various complement-free test sera containing anti-blood group antibodies such as anti-A, anti-B and anti-D. Red blood cells presenting surface antigens of a particular specificity will be bound by antibodies specific for that antigen. Further reaction by additional antibodies will lead to formation of bridges between adjacent cells and eventually an interlocked mass of erythrocytes joined to each other. Such a reaction is visually detectable and evaluation can be automated by spectrophotometric means.

U.S. Patent No. 3,990,850 to Friedman, et al. discloses test cards for blood typing wherein serum containing antibodies specifically reactive with blood group antigens is dried onto a substrate such as plasticized nitrocellulose having water-impermeable and water wettable properties. Testing for blood types is conducted by means of liquid phase agglutination testing wherein the antiserum is reconstituted by addition of distilled water and a blood sample is added to and mixed with the antiserum. Agglutination of the blood sample is detectable visually.

Antibodies reactive with particular antigens are often incapable of agglutinating erythrocytes because of repulsive forces caused by the negative surface potential surrounding erythrocytes. IgM antibodies are sufficiently large to span the gap between the mutually repelling cells, but smaller IgG antibodies are frequently incapable of bridging the gap. In situations where IgG antibodies do not spontaneously cause agglutinization, such a reaction must be induced in a number of ways which can include (1) mild centrifugation to overcome the repulsive forces and allow for immune crosslinkage, (2) suspension of the test solution in an anisotropic medium such as albumin (22%) or polyvinylpyrrolidone (PVP), or (3) through use of the "Coombs reaction" whereby antihuman antiglobulins are used to link the antibody molecules to one another.

Cell lysis tests do not rely on agglutination, but rather determine whether a specifically reactive antibody added to a red blood sample together with serum complement will cause cell lysis. The association of serum complement with an antibody bound to a cell membrane results in disruption of the cell membrane, release of the intracellular contents and cell death.

Both the agglutination and cell lysis methods may be used quantitatively as well as qualitatively and are subject to automation. Both methods may also be used to "back test" blood sera to confirm tests conducted on red blood cells. Such back testing is possible because blood sera contains antibodies complementary to antigens presented by its associated red blood cells, that is specific for major blood group antigens not present on its associated blood cells.

Substantial limitations exist with respect to use of the conventional liquid phase methods for blood typing. Evaluation of agglutination test results often requires the subjective judgment of experienced personnel to distinguish agglutination of cells due to antigen-antibody reaction from non-specific aggregation in which unrelated forces cause some degree of clumping. While automated equipment which can provide more quantitative results is available, it is expensive (costs estimated at from $115,000 to $420,000) and hence not readily available in numerous clinical situations (e.g., physician's offices, blood banks, emergency rooms, military operations) where a rapid, simple and accurate test is desired. Further, while methods based on cell lysis are often of use in evaluations of tissue types, they are often of limited use when applied to human erythrocytes either because the cell membrane antigen-antibody complex reacts inefficiently with the serum complement or because the antibody concentration must be limited to prevent intense agglutination that will mechanically interfere with complement mediated lysis.

Considerable advances have been made with respect to solid phase serologic procedures. U.S. Patent No. 4,275,053 issued to Rosenfield, et al. discloses solid-phase blood typing procedures whereby a monolayer of red blood cells is covalently bound to the walls of a plastic tube. The monolayer is then contacted with a serum sample and immunoadsorption of the antibodies present in the sample by the bound red blood cells occurs when the antibodies are reactive with antigens presented by the cell membranes. The antibodysensitized monolayer of blood cells can then bind a second layer of blood cells carrying complementary antigen in an agglutination reaction. If the immunological type of both the cell monolayer and the antibody layer are known, the formation or non-formation of a second cell layer can be used to assay the immunological type of the cells forming the second layer. Conversely, if the immunological specificity of the first cell layer is known, the ability to form a second cell monolayer with the same cells can be relied on as a means for determining whether or not there had been formed a layer of antibodies immunosorbed by antigens onto the first cell layer. Densitometric techniques may be used to provide a quantitative evaluation of the results.

Rosenfield, et al. discloses the use of polystyrene and polyurethane tubes as a solid matrix for the binding of erythrocytes. The use of other polymers which present, on their surface, reactive groups which will allow direct covalent bonding of blood cells to the matrix such as glutaraldehyde, cyanogen bromide, amino and carboxyl groups is also suggested. The surfaces are treated with fibroinogen and polylysine solutions in order to irreversibly bind erythrocytes to the substrate surface. The use of other binding agents such as phytohemaglutinin, concanavalin A and pokeweed mitogen is also suggested to assist binding of erythrocytes to the substrate surface. Antibodies are then incubated with the bound monolayer of erythrocytes. In forming the second erythrocyte monolayer, merely applying a cell suspension and allowing it to settle by gravity, even for 25 minutes, is usually inadequate to provide antibody cross-linkage with the antibodies and the first monolayer. It is therefore necessary to augment formation of the second monolayer with treatments using agents such as PVP or preferably protamine sulfate in addition to incubating the reaction mixture for up to 25 minutes.

U.S. Patent No. 4,252,538 issued to Barr discloses blood typing devices and procedures similar to those of Rosenfield, et al. Barr discloses use of plastic substrate materials (such as polycarbonate, polystyrene, acrylics, and others) coated with bovine serum albumin (BSA). The BSA is then coated with chromic chloride ($CrCl_3$) which serves to bind erythrocytes to the BSA and hence to the substrate surface.

Lee, et al., PCT International Application WO 86/05591 (European Application. 86901901.8) disclosing a method for the immunological determination of a biological sample, has a priority date of March 19, 1985 and a publication date of September 25, 1986 and thereby falls within the terms of Article 54(3) EPC. Such application discloses the binding of one or more purified antibodies specific to antigenic determinants of red blood cells to a solid support, incubation of an unknown blood sample with the solid support on which the antibodies are bound, rinsing the support to remove unbound red cells, and detecting the red color of the red cells bound to the support. Said antibodies can also be bound in a certain array on the solid support. Among possible solid supports, nitrocellulose has been cited.

Bayer, et al., PCT International Application WO85/01354, published March 28, 1985, discloses solid phase methods for blood group typing wherein known antibodies are attached to a solid surface and proteolytic enzyme activated red blood cell samples are presented for an immunological reaction. [See, also, Plapp, et al., Laboratory Management, (December, 39-47 1984).] Red cells adhering to the surface provide an indication of the presence of particular antigens. A "back type" procedure is also disclosed wherein synthetic or purified antigens are attached directly to a solid substrate. The surface is then contacted with blood serum of unknown specificity to permit antibodies therein to undergo immunological reaction with the previously attached antigens. A second solution of erythrocytes presenting known antigens is then used as an indicator means wherein binding of the second layer of erythrocytes indicates the antigenic specificity of the unidentified antibodies.

Solid substrates disclosed by Bayer, et al. include beads, test tubes, sheets, strips, and microtiter plates all made of polystyrene or polyvinylchloride. Preferably used are polystyrene microtiter plates having U-shaped wells. Antibody solutions are immobilized on the solid surfaces by long term incubation or heat treatment with care taken to allow for uniform distribution of antibodies across the well surface. It is noted that anti-D antibody does not bind Rh positive red blood cells strongly enough to withstand centrifugal forces applied in the test procedure so it is considered desirable to immunobind anti-D to antihuman immunoglobulin (IgG) which is first immobilized on the surface.

Bayer, et al. requires that the blood being tested in the forward blood typing procedure be "activated" by treatment of erythrocytes with a proteolytic enzyme to promote binding of the blood cells by the antibody layer. Results are read for both the forward and back type tests by allowing the blood component to incubate in the microtiter wells. Positive reaction of the red blood cells with the antibody layer is indicated by a uniform effacement of red blood cells over the entire concave bottom surface of the well upon centrifugation. A negative reaction is characterized by all of red blood cells settling to the bottom of the well into a small "button" in the center. Alternatively, it is proposed that after the red blood cells have been allowed to adhere, the microtiter plate well solid surfaces may be washed with a saline solution to remove unreacted red blood cells. It is also proposed that the plate can be inverted and subjected to mild centrifugation to remove "buttons" of red blood cells. In these alternative procedures, a negative reaction would be indicated by a clear well, devoid of red blood cells. Plapp, et al. propose that in the solid phase test, determination of the presence of a "negative" colored "button" of erythrocytes can be distinguished from a "postiive" effacement of erythrocytes over a broader area by means of automated devices costing on the order of $20,000.

Of interest to the background of the invention are the disclosures of Gordon, EPO Application 63810, published November 3, 1982, relating to devices for effecting multiple immunological analyses on a single sample and to methods for their use. The devices consist of a porous solid support containing a preselected array of delimited adsorption areas of antigens, antibodies, or both, wherein residual adsorption sites on the substrate are saturated by protein blocking agents such as bovine serum albumin which do not cross-react with the antigens or antibodies employed in the assay. The porous supports are disclosed to be materials with sufficient surface porosity to allow access by antibodies as well as suitable surface affinity to bind antigens. Such materials can be (1) natural polymeric carbohydrates and their synthetically modified derivatives including nitrocellulose; (2) natural polymers containing nitrogen such as gelatin; (3) natural

4

EP 0 223 978 B1

hydrocarbon polymers such as latex; (4) synthetic polymers including polyethylene, polystyrene, polyvinylchloride, polyesters, polyamides and polyurethane; (4) inorganic porous materials such as clays as well as (5) mixtures or copolymers of the above classes. The materials are disclosed to be used in suitable shapes such as films, sheets, plates and cylinders but most preferably sheets 0.l mm thick with pore size between 0.l5 and l5 $\mu$m.

Antigens or antibodies are applied to the porous solid support by direct contact followed by incubation with the blocking agents. Assays for detection of unknown antigens or antibodies are then carried out through use of radioactive or enzyme linked indicator antibodies which can also be antihuman or antianimal immunoglobulin antibodies. Results of the multiple assays are then determined by detection of multiple indicator antibodies.

Despite the advances in the art, there still exists a need in the art for rapid, simple, accurate and inexpensive methods and devices to conduct blood typing tests.

Optimally, solid phase immunological devices employed in blood typing should be inexpensively fabricated using minimum amounts of antibodies, blood group substances or other reagents (e.g., binding and blocking reagents) antibodies, blood group substances with simple, uniform means available for distribution of these substances. Blood sample materials tested should be subjected to minimum or no pretreatment. Assay procedures should be rapid and should not require multiple sample or reagent manipulations or the use of apparatus (shakers, centrifuges and the like) to develop desired reactions. Indicators of assay results should be readily stabilized to allow for delayed "reading" of assays. Actual reading of assay results should be unambiguous and not require use of automated apparatus.

BRIEF SUMMARY

The present invention provides a new, simple and inexpensive device and methods of its use for determining blood type classifications from blood or serum samples. The device utilizes a minimum of antibody material, requires no additional apparatus or instrumentation for its use and is particularly useful in physicians' offices, blood banks, emergency rooms, military operations and in underdeveloped areas. The test device comprises (a) a porous solid substrate having an average pore size of 0,025 - 7,0 $\mu$m; and (b) one or more essentially pure antibodies specifically reactive with erythrocyte antigenic determinants characterizing blood groups wherein the antibodies are bound to the porous solid substrate in an array of delimited adsorption areas of differing specificities such that they are capable of binding erythrocytes for which they are specifically reactive so as to produce detectable color signal within the delimited area in contrast to areas on the surface of the substrate where no antibodies are bound.

The test device may be used to determine the blood type classification of a sample by (a) incubating the test device with a sample of blood, the type of which is to be determined; (b) removing the test device from the sample and rinsing the device; and (c) detecting a color signal from erythrocytes bound to antibodies which are themselves bound to the solid substrate. A single test determination can be performed by hand in less than one minute.

Also provided is an alternative test device for determining blood type classifications through serum "back typing" procedures. The test device comprises (a) a porous solid substrate having an average pore size of 0,025 - 7,0 $\mu$m; and (b) one or more blood group substances providing antigenic determinants characteristic of specific blood groups wherein the blood group substances are associated with the solid substrate in an array of delimited adsorption areas such that they are capable of binding antibodies from a serum sample which are themselves capable of binding erythrocytes for which they are specifically reactive so as to produce detectable color signals. The alternative test device may be used to determine blood group classifications through serum "back typing" methods comprising (a) incubating the alternative test device with a sample of whole blood or serum isolated from whole blood; (b) removing the test device from the sample and rinsing the device; and (c) contacting the test device with a mixture of erythrocytes comprising erythrocytes presenting antigenic determinants characteristic of various blood groups; and (d) detecting a color signal from erythrocytes bound through the serum antibodies to the blood group substance associated with the solid substrate. Back typing methods according to the invention detect major blood group antibodies present in the blood serum and indicate by implication the blood types complementary to these.

DETAILED DESCRIPTION

Description of the Porous Solid Substrate

5

Generally, the porous solid substrates useful with the present invention may be any material with sufficient surface porosity to allow access by immunoglobulins and a suitable surface affinity to allow binding of such antibodies, preferably without resort to reagents and conditions needed for covalent binding. In a like manner, suitable porous substances will allow access and binding of antigens employed in back type assay devices. Such materials can include fibrous materials including woven and nonwoven fabrics. Microporous structures are generally preferred, but materials with gel structure in the hydrated state can be used as well. As to their chemical nature they may be:

(A) Natural polymeric carbohydrates and their synthetically modified, cross-linked or substituted derivatives, such as (a) agar, agarose; cross-linked alginic acid; substituted and cross-linked guar gums, cross-linked dextran polymers and starches; (b) regenerated celluloses; cellulose esters, especially with nitric acid and carboxylic acids; mixed cellulose esters, cellulose ethers, especially with lower aliphatic alcohols.

(B) Natural polymers containing nitrogen, such as proteins and derivatives, e.g., cross-linked or modified gelatin.

(C) Natural hydrocarbon polymers, such as latexes and rubbers.

(D) Synthetic polymers which can be prepared with suitably porous structures, such as (a) vinyl polymers, such as polyethylene, polypropylene, polystyrene, polyvinylchloride, polyvinylacetate and its partially hydrolysed derivatives, polyacrylates, polyacrylamides, polymethacrylates; (b) copolymers and terpolymers of the above vinyl monomers among themselves and with other monomers; (c) polycondensates, such as polyesters, polyamides; (d) addition polymers, such as polyurethanes or polyapoxides.

(E) Inorganic materials which can be prepared in a suitably porous form, such as sulfates or carbonates of alkaline earth metals and magnesium, e.g., barium sulfate, calcium sulfate, calcium carbonate, magnesium carbonate, or silicates of alkali and alkaline earth metals and/or aluminum and/or magnesium, and aluminum or silicon oxides or hydrates, such as clays, alumina, talc, kaolin, zeolite, silicagel, glass. These materials can be used as such or as fillers in one of the above polymeric materials.

(F) Mixtures or copolymers of the above classes, such as graft copolymers obtained by initiating polymerization of synthetic polymers on a pre-existing natural polymer.

A porous solid substrate material which is preferred for use with the present invention is a microporous cellulose ester, for example, an ester of cellulose with an aliphatic carboxylic acid, such as an alkane carboxylic acid, having from 1 to 7 carbon atoms, e.g., acetic acid, propionic acid, or any of the butyric acids or valeric acids. Especially preferred is porous substrate material made from nitrocellulose, by which term any nitric acid ester of cellulose is intended, if desired, in mixture, with any of the said carboxylic acid cellulose esters. Thus, pure nitrocellulose esters can be used as consisting of an ester of cellulose having approximately 3 nitric groups per 6 carbon atoms. Most preferred is a nitrocellulose material under the trade name "Millipore" (Millipore Corp., Bedford, Massachusetts) which has a pore size of 0.45 $\mu$m.

All these materials may be used as such in suitable shapes, such as films, strips or sheets. They may also be coated onto or bonded or laminated to appropriate inert carriers, such as: paper, glass, plastic, metal or fabrics. The porous solid substrate is preferably in the form of strips of thickness in the range from about 0.01 mm to about 0.5 mm, and most preferably of about 0.1 mm. Of course, the thickness of the porous substrate will affect the amount of antibody solution that need be applied to the substrate to produce a spot of a given surface area. The pore size may vary within wide limits, but is preferably between about 0.025 $\mu$m and about 7 $\mu$m, especially between about 0.15 $\mu$m and about 4 $\mu$m and most preferably about 0.45 $\mu$m. It should be noted that the erythrocytes have diameters of approximately 7 $\mu$m and may become physically (i.e., non-immunologically) trapped within the pore structure of substrates with pore sizes of slightly smaller than that size or larger.

Description of the Antibodies

As employed herein with respect to specific blood type antibodies useful in practice of the invention, the term "essentially pure" shall designate preparations of IgM or IgG antibodies, or mixtures thereof, which are essentially free of association with antibodies capable of binding to erythrocytes in an immunological reaction not based on specified blood group antigens and are essentially free of association with materials capable of interfering with an antigen-antibody reaction involving antigenic determinants characteristic of blood type. By way of illustration, "essentially pure" is applicable to describe an essentially homogeneous preparation of polyclonal anti-A or anti-B or anti-D antibodies purified from serum by affinity purification techniques employing corresponding A, B or D antigens as the sole absorbant. The term is correspondingly applicable to describe essentially homogeneous preparations of monoclonal anti-A or anti-B or anti-D antibodies purified from cell culture media or ascites fluid by affinity purification or by chromatographic

6

techniques based, e.g., on absorbants specific for IgM or IgG type immunoglobulins.

Generally, the immunoglobulins useful with the present invention may be those immunoglobulins specifically reactive with various antigenic determinants characteristic of particular blood groups. The antibodies may be IgM antibodies, IgG antibodies or mixtures thereof. Antibodies specifically reactive with antigens characterizing the various major and minor blood group systems are contemplated by the present invention. Such major blood group systems include the ABO, D (Rh), Lewis, I, Kell, P, Mn, Lutheran, Kidd and Duffy systems. Other blood group antigens and systems include the $Xg_a$, Dombrock, Cartwright, Langereis, Diego, $S_c$, Colton, Wright, Vel, Gerbich, HD-Pr-Sp, Stoltzfus, Bg Stirling, York, Gursha, Gregory, Holley, Knops-Helgeson, MPD, Vennerra, Dp, El, JMH, T, Cad and Sd systems and antigens.

The antibodies may be polyclonal or monoclonal and are commercially available or may be obtained by mouse ascites, tissue culture or other techniques known to the art. A typical description of hybridoma procedure for the production of monoclonal antibodies may be found in Wands, J.R., and V.R. Zurawski, Gastroenterology 80:225 (l981); Marshak-Rothstein, A., et al.; J. Immunol. 122:2491 (1979); Oi, V.T. and L.A. Herzenberg, "Immunoglobulin Producing Hybrid", Mishell, B.B. and S.M. Shiigi (eds.) Selected Methods in Cellular Immunology, San Francisco: W.H. Freeman Publishing, 1979; and U.S. Patent No. 4,515,893 issued May 7, 1985 to Kung, et al. Mixtures of monoclonal antibodies of differing antigenic specificities or of monoclonal antibodies and polyclonal antibodies may be desired. Regardless of the particular source or type of antibodies, however, they should be essentially pure as defined above. The antibodies may be purified by column chromatographic or other conventional means but are preferably purified according to known affinity purification techniques.

Monoclonal antibodies presently preferred for use with the invention include mouse monoclonal anti-A immunoglobulin (designated AH8-39 from Chem Bio Med, Ltd. (Edmonton, Alberta)); mouse monoclonal anti-B immunoglobulin (designated AH8-33 from Chem Bio Med, Ltd. (Edmonton, Alberta)); and human polyclonal anti-D ($Rh_o$) antibody which is obtainable from Dade Division, American Hospital Supply Corp. or BCA Division, Cooper Biomedical. These commercially available antibodies, however, must be made essentially pure as by affinity purification, if such was not done as a part of the commercial preparation, before they may be used with the present invention.

Description of Blood Group Substances

Blood group substances useful with the alternative back type test device of the present invention include those materials, whether natural or synthesized, which present antigenic determinants characteristic of the various blood types when bound to the porous solid substrate. The substances include blood cells presenting blood type antigens including red blood cells (erythrocytes) but may also include cells of other functionalities and is not limited to blood cells. Erythrocyte "ghosts" which are red blood cells which have been lysed and had their hemoglobin removed are particularly useful. Moreover, it is contemplated that blood group surface antigens may be isolated from the cell membranes upon which they are normally presented and may be presented on other materials. It is further contemplated that polypeptide and polysaccharide antigens may be chemically synthesized which duplicate the antigenic determinants characteristic of blood group antigens. Antigens presented by the blood group substances include those associated with the ABO major blood group although others are contemplated by the present invention.

EXAMPLE 1

In this example, a description is provided of a procedure useful in the affinity purification of polyclonal anti-D antibody.

Whether the antibodies to be used with the invention are monoclonal, polyclonal, obtained commercially or produced specifically for the invention, it is preferred that they be purified according to affinity purification techniques such as this one. A sufficient volume of whole blood is added to a quantity of anti-D (Rh) immunoglobulin to bind all of the antibody in one hour at 37°C. The bound red blood cells are then harvested by centrifugation and to one volume of the red blood cells is added nine volumes of 0.1% digitonin in saline (0.9% NaCl solution). The mixture is shaken vigorously for one minute and the resulting membranes are then harvested and washed by centrifugation until a white pellet is obtained.

One volume of cell membranes is then mixed with one volume of a 6% albumin solution and two volumes of ether and shaken vigorously for one minute. The mixture is then incubated at 37°C for 15 minutes. From this mixture, the albumin fraction which contains the anti-D immunoglobulin may be isolated by centrifugation. Sufficient protein A-sepharose is then added to bind the anti-D immunoglobulin in one hour at room temperature. The protein A-sepharose/immunoglobulin conjugate is then poured into a gel filtration column and is washed with PBS to remove unbound material. The anti-D immunoglobulin is eluted with PBS (pH 3.0). The final pH of the immunoglobulin is then adjusted to 6.5 to 7.5 and the material is stored until use.

EXAMPLE 2

In this example, a description is provided of a column chromatography technique suitable for purification of the antibodies useful in practice of the invention. It is known that IgG antibodies useful in practice will bind strongly to protein A, a cell wall protein isolated from Staph. aureus, while IgM antibodies will bind strongly with protamine. In this example, protamine which is covalently bound to Sepharose is used to selectively adsorb IgM from mouse ascitic fluid. Similar processes can be utilized to purify IgG antibodies utilizing columns packed with protein A material.

Protamine is coupled to Sepharose 4B using cyanogen bromide treatment, or such material may be obtained commercially. A column is then packed with the protamine beads and is equilibrated with a phosphate/saline buffer solution (Buffer A) comprising a solution made up of 0.03M $KH_2PO_4$, 0.026M NaCl, and a solution made up of 0.05.% $NaN_3$ and 0.03M $Na_2HPO_4$, 0.026M NaCl and 0.05% $NaN_3$ mixed in quantities such that they have a pH of 7.4. Five column volumes of Buffer A is then run through the chromatography column.

The ascites fluid to be purified is diluted with 4 parts of Buffer A solution and is applied to the column. The IgM will be selectively adsorbed onto the column by the bound protamine and will not elute along with the buffer solution. The selectively adsorbed IgM may then be eluted by using a different phosphate/saline buffer solution (Buffer B) which comprises a solution made up of 0.08M $KH_2PO_4$, 1.1M NaCl, and 0.05% $NaN_3$ and a solution made up of 0.08M $Na_2HPO_4$, 1.1M NaCl and 0.05% $NaN_3$ mixed in quantities such that they have a pH of 7.4. Ten column volumes of Buffer B is then eluted through the column in order to extract the desired IgM.

The eluate from the above procedure contains the desired IgM. The antibody containing eluate is then applied to Sepharose-400 sizing beads and Tris-Glycine buffer is eluted through the column. Fractions of the eluate are then evaluated for presence of the desired IgM and the desired fraction is isolated and stored.

EXAMPLE 3

A. Manufacture of the Forward Typing Device

The essentially pure antibodies should be applied to the porous solid substrate in an array of delimited adsorption areas such that separate immunological testing can be conducted with respect to each adsorption area. In applying the antibodies to the porous solid support a variety of means may be practiced. The immunoglobulin may be applied to the porous substrate by manual means such as capillary tubes, pipettes or syringes or by means of a spray aided by gaseous or liquid propellants. Where the immunoglobulin is applied by means of a spray, a template or applicator miniaturized by means of procedures such as are known in the microelectronics art may be used in conjunction with known lithographic techniques.

The antibody may be applied so as to provide any suitable geometry such as dots, lines or spots. An array of parallel lines can be cut into a number of strips each comprising a number of segments each containing an antibody of different specificity. Such strips would readily lend themselves to mass production techniques. The array can include from one to a great number of antibodies but they are preferably applied in small volumes because of their scarcity and expense.

It is preferred that antibody solutions be applied in volumes smaller than 1 $\mu$l with volumes of about 0.5 $\mu$l particularly preferred for producing spots of about 1-2mm in diameter. Spots of this size are preferred for devices relying upon visual examination of positive color signals. Smaller volumes of antibody solution may also be used but such volumes may produce smaller spots which require evelution by spectrophotometric means. Where it is contemplated that devices of the present invention be evaluated by nonvisual means such as with a reflectance spectrophotometer it is preferred that volumes smaller then 0.5$\mu$l be applied so as to further reduce the amounts of antibody materials required for fabrication of the devices of the invention.

Concentrations of antibodies applied in solutions are preferably in the range of about 100-150 $\mu$g of IgM per ml of solution when applied to the nitrocellulose sheets of the present invention. Most preferred are antibody concentrations in the range of about 120-140 $\mu$g of IgM per ml of solution. Greater concentrations can be applied but may not serve to increase the binding efficiency of the spots. Lesser concentrations may also be applied with the effect that density of positive color signals will be diminished. While it is expected that lesser antibody concentrations may reduce positive color signal intensity to the extent of hindering visual evaluation of test results, it is nevertheless contemplated that reduced color signal intensity will not unduly hinder spectrophotometric evaluations of the devices of the invention.

The following is a description of the preparation of a specific device for rapid ABO and D blood typing.

Nitrocellulose filter paper sheets (Millipore Corporation) having a thickness of 0.1 mm and an average pore diameter of 0.45 $\mu$m were cut into strips and mounted on a rigid plastic support using a suitable adhesive. Onto each strip was appplied the following: 70.5 ng of affinity purified mouse monoclonal anti-A IgM antibody obtained from Chem Bio Med, Ltd. (Edmonton, Alberta); 60.0 ng of affinity purified mouse monoclonal anti-B IgM antibody obtained from Chem Bio Med, Ltd. (Edmonton, Alberta); and human polyclonal anti-D IgG antibody obtained at a titer of 1:30,000 as a reagent for slide and tube testing from Dade Division, American Hospital Supply, or from BCA Division of Cooper Biomedical which was affinity purified according to the procedure of Example 1. The anti-A antibody was diluted in a PBS solution containing 1% sucrose to a titer of 1:4000 (141 $\mu$g/ml) and was applied to the nitrocellulose strip in 1-2 mm diameter spots at a rate of 0.5 $\mu$l per spot by means of a Hamilton microtiter automatic syringe. The anti-B antibody was diluted in a PBS solution containing 1% sucrose to a titer of 1:4000 (120 $\mu$g/ml) and was applied to the nitrocellulose strip in 1-2 mm diameter spots at a rate of 0.5 $\mu$l per spot by means of an automatic syringe. The affinity purified anti-D antibody was diluted to a titer of 1:100,000 and was applied to the nitrocellulose strip in 1-2 mm diameter spots at a rate of 0.5 $\mu$l per spot by means of an automatic syringe. The strips were then allowed to dry and were stored desiccated until use.

The present invention is noteworthy in that when the preferred nitrocellulose substrate is used, there is no necessity to incubate the antigen or antibody array with protein blocking agents such as bovine serum albumin as is required by Gordon. Such incubation may even provide detrimental to the devices of the present invention. Therefore, after application of the antibodies, the test devices are allowed to dry at room temperature and are stored desiccated until use.

B. Forward Blood Typing Procedure

The following "forward" blood typing procedure is used to type blood according to the invention. Blood is collected, most preferably whole blood, and is treated with anticoagulant. Acid citrate dextrose (ACD) is the anticoagulant of choice but other anticoagulants may be used such as EDTA or heparin. Blood cells from a clot tube may be resuspended in saline solution for use with the present invention and capillary blood may also be used.

The test is conducted by contacting the test device with a sample of the blood to be tested preferably by immersion, although other methods such as spotting the blood onto the strip are acceptable. The blood is contacted with the test strip for from 1 to 30 seconds but preferable for only 2 to 5 seconds. The test strip is then immediately washed gently in a normal saline (0.9% NaCl) solution either by rinsing or preferably by dipping and gentle agitation for 10-15 seconds at room temperature.

The test strip should then be read within two minutes of the reaction. Alternatively, the results can be fixed on the test device by dipping the device into a solution of methyl alcohol, acid or other appropriate fixative. A positive reaction will be signaled by a red spot against the background of the porous substrate. The red spot can be detected visually or by an instrument such as a reflectance spectrophotometer. The red spot is created by the attachment of erythrocytes to the antibody localized on the membrane. Blood cells of the blood type A will stick to spots containing A antibody, blood cells of blood type B will stick to spots containing B antibody and blood cells of type D (Rh$_o$) will stick to spots containing D (Rh) antibody. Where a test device contains 3 spots containing A, 8 and D (Rh) antibodies blood type results can be read and interpreted according to the following protocol:

## TABLE I

| A Spot | B Spot | D (Rh) Spot | Interpretation |
|:------:|:------:|:-----------:|:--------------:|
| + | − | − | A-negative |
| + | − | + | A-positive |
| − | + | − | B-negative |
| − | + | + | B-positive |
| + | + | − | AB-negative |
| + | + | + | AB-positive |
| − | − | − | O-negative |
| − | − | + | O-positive |

Analysis for other major and minor blood groups and blood type antigens can be carried out the same way. Such devices can also include spots for use as positive and negative controls. As a positive control, antihuman red cell immunoglobulin can be spotted to the test device to present a color signal for the presence of human erythrocytes. A negative control can be provided by spotting of a non-erythrocyte specific immunoglobulin.

### EXAMPLE 4

In this example, blood of various ABO (D) types was tested by means of test strips fashioned and used according to the methods described in Example 3. As a reference, the samples were also tested by standard agglutination techniques. The method of the invention agreed with the reference method as to the ABO classification of the blood samples in 100% of 171 cases. With respect to D (Rh) classification, the method of the invention agreed with the results of the agglutination method in 100% of 166 cases. With respect to detection of the $D^{\mu}$ variant form, however, the method of the invention detected only 4 samples as positive for $D^{\mu}$ while the reference method identified 7 as positive. The method of the invention identified 3 of the $D^{\mu}$ positive samples as D-negative.

## TABLE II

| Test Strips of the Invention | Reference Method (Agglutination) | | | | | | |
|---|---|---|---|---|---|---|---|
| | A | B | AB | O | D(+) | D$^\mu$ | D(−) |
| A | 52 | 0 | 0 | 0 | 0 | 0 | 0 |
| B | 0 | 14 | 0 | 0 | 0 | 0 | 0 |
| AB | 0 | 0 | 13 | 0 | 0 | 0 | 0 |
| O | 0 | 0 | 0 | 92 | 0 | 0 | 0 |
| D(+) | 9 | 0 | 0 | 0 | 144 | 0 | 0 |
| D$^\mu$ | 0 | 0 | 0 | 0 | 0 | 3 | 0 |
| D(−) | 0 | 0 | 0 | 0 | 0 | 4 | 22 |

The present invention also provides an alternative device for the typing of blood serum antibodies. The device is also capable of "back typing" a blood group because of the complementary relationship between blood cell type and serum antibody type. The alternative device comprises the porous solid substrate of the forward typing device to which blood group substances are bound in an array of delimited adsorption areas. Alternatively, it is contemplated that the blood group substances may be bound indirectly to the porous substrate by means of an immunological reaction with antibodies which are themselves covalently bound to the substrate. Such antibodies could be bound to the substrate according to the methods of the direct typing device.

In one embodiment, blood group substances presenting antigens of the same antigenic specificity for which the serum will be back typed are applied to a porous solid substrate according to the methods for constructing a direct typing device. The blood group substances thus present an antigenic site against which serum antibodies of particular specificities are reactive.

To illustrate, a device to detect anti-A serum antibodies can be constructed by applying a blood group substance presenting an A antigen such as lysed A-negative red blood cells to a porous solid substrate. A drop of the serum sample to be tested is then applied to the test device under conditions appropriate for an immunological reaction. If anti-A antibodies are present in the serum sample, they will bind to the blood group substances. In the final step, unlysed type A red blood cells still presenting their red color are applied to the test device under conditions appropriate for an immunological reaction. If anti-A antibodies were present in the serum to be tested, they will be bound to the blood group substances on the test device. They will also serve to bind the red blood cells to the test device. After rinsing away any unbound red blood cells by method of Example 3, the detection of a red color signal on the porous substrate indicates that red blood cells are bound to the serum antibodies and is a positive indication for type A serum antibodies. This in turn indicates that the corresponding blood type is not type A. The red color signal can be detected visually or by instruments such as a spectrophotometer.

Blood group substances which present more than a single blood group antigenic determinant can complicate back type analysis because of their ability to react ambiguously with more than one specificity of serum antibody. For this reason, blood group substances presenting simply type A antigens or type B antigens are preferred over those presenting multiple antigens such as type AB erythrocyte ghosts. Nevertheless, any ambiguities in analysis can be cured by a careful selection of the blood types present in the red blood cells applied to visualize the serum antibodies and by running an array of test spots with a variety of blood group substances bound to the porous substrate which are sensitive to specific antibodies. A variety of combinations of blood group substance specificities, base antibody specificities and labelling erythrocyte specificities can be designed so as to detect the presence of any combination of serum antibodies and thus blood type.

**Claims**

1. A test device for determining the blood type of a blood sample comprising:
(a) a solid substrate;
(b) one or more essentially pure antibodies specifically reactive with erythrocytic antigenic determinants characteristic of a blood type, each antibody being bound to said substrate in a delimited adsorption area and being capable of immunologically binding to the surface of said substrate blood sample erythrocytes for which the antibody is specifically reactive so as to produce a detectable color signal within the delimited area in contrast to areas on the surface of said substrate where no blood type antibodies are bound, characterized in that said solid substrate is porous, having an average pore size of 0.025 to 7.0 $\mu$m.

2. The test device of claim 1 wherein said substrate is a nitrocellulose sheet.

3. The test device of claim 1 wherein the antibodies are noncovalently bound to said substrate.

4. The test device of claim 1 wherein the antibodies are affinity purified antibodies.

5. The test device of claim 1 wherein the antibodies are selected from the group consisting of anti-A, anti-B and anti-D antibodies.

6. The test device of Claim 2, wherein the antibodies are one or more essentially pure antibodies selected from the group consisting of affinity purified monoclonal anti-A antibodies, affinity purified monoclonal anti-B antibodies, and affinity purified polyclonal anti-D antibodies, each antibody being noncovalently bound to the sheet.

7. The test device of claim 6 wherein the nitrocellulose sheet has a pore size of about 0.45 $\mu$m.

8. A method for determining the blood types of a blood sample comprising:
(a) incubating the test device of claim 1 or 6 with a sample of blood, the type of which is to be determined;
(b) rinsing the test device ;
(c) detecting a color signal from erythrocytes immunologically bound to antibodies bound to the porous solid substrate.

9. A test device for determining the blood type of a blood sample comprising:
(a) a solid substrate;
(b) a blood group substance providing antigenic determinants characteristic of a specific blood type, such blood group substance being bound to the solid substrate such that it is capable of binding serum antibodies from a serum sample which antibodies are themselves capable of immunologically binding erythrocytes with which the antibodies are specifically reactive, characterized in that said solid substrate is porous, having an average pore size of 0.025 to 7.0 $\mu$m, and said blood group substance can further be blood group substances of more than one specific blood type, each blood group substance of a specific blood type being bound to the solid substrate in a delimited adsorption area, so as to produce a detectable color signal within the delimited area in contrast to areas on the surface of said substrate where no blood group substances are bound.

10. The test device of claim 9 wherein the porous solid substrate is a nitrocellulose sheet.

11. The test device of claim 9 wherein the blood group substance is selected from a group consisting of blood group substances providing an antigenic determinant characteristic of blood types A or B.

12. The test device of claim 9 wherein the blood group substance is an oligosaccharide.

13. The test device of claim 9 wherein the blood group substance is a lysed erythrocyte membrane of a characteristic blood type specificity.

14. A method for determining the blood type of a blood sample comprising:
(a) incubating the test device of claim 9 with a sample of whole blood or serum isolated from whole blood, the type of which is to be determined;
(b) rinsing the test device;
(c) contacting the test device with a mixture of erythrocytes comprising erythrocytes presenting antigenic determinants characteristic of the blood types to be tested.
(d) detecting a color signal from erythrocytes bound through the serum antibodies to the blood group substance bound to the solid substrate.

15. The method of claim 14 wherein the mixture of erythrocytes comprises erythrocytes of type A and erythrocytes of type B.

16. The method of claim 14 wherein the mixture of erythrocytes comprises erythrocytes of type AB.

## Patentansprüche

1. Testeinrichtung zur Bestimmung des Bluttypus einer Blutprobe, welche Testeinrichtung umfaßt:
(a) ein festes Substrat;
(b) einen oder mehrere, im wesentlichen reine Antikörper, welche mit Erythrozyten-Antigendeterminanten, die für einen Bluttypus kennzeichnend sind, spezifisch reaktiv sind, wobei jeder Antikörper an das Substrat in einer abgegrenzten Adsorptionsfläche gebunden ist und befähigt ist, in der Blutprobe vorhandene Erythrozyten, mit welchen der betreffende Antikörper spezifisch reaktiv ist, immunologisch an die Oberfläche des Substrates zu binden, um innerhalb der abgegrenzten Fläche - im Gegensatz zu Flächenteilen auf der Oberfläche des Substrates, auf welchen keine Bluttypen-Antikörper gebunden sind - ein feststellbares Farbsignal zu erzeugen, dadurch gekennzeichnet, daß das feste Substrat porös ist, nämlich eine mittlere Porengröße von 0,025 bis 7,0 $\mu$m aufweist.

2. Testeinrichtung nach Anspruch 1, wobei das Substrat ein Nitrozelluloseblatt ist.

3. Testeinrichtung nach Anspruch 1, wobei die Antikörper an das Substrat nicht-kovalent gebunden sind.

4. Testeinrichtung nach Anspruch 1, wobei die Antikörper durch Affinitätschromatographie gereinigte Antikörper sind.

5. Testeinrichtung nach Anspruch 1, wobei die Antikörper aus einer Gruppe ausgewählt sind, welche aus Anti-A-, Anti-B- und Anti-D-Antikörpern besteht.

6. Testeinrichtung nach Anspruch 2, wobei die Antikörper ein oder mehrere, im wesentlichen reine Antikörper sind, welche aus einer Gruppe ausgewählt sind, die aus durch Affinitätschromatographie gereinigten monoklonalen Anti-A-Antikörpern, durch Affinitätschromatographie gereinigten monoklonalen Anti-B-Anitkörpern und durch Affinitätschromatographie gereinigten monoklonalen Anti-D-Antikörpern besteht, wobei jeder Antikörper nicht-kovalent an das Blatt gebunden ist.

7. Testeinrichtung nach Anspruch 6, wobei das Nitrozelluloseblatt eine Porengröße von etwa 0,45 $\mu$m aufweist.

8. Verfahren zum Bestimmen der Bluttypen einer Blutprobe, welches Verfahren umfaßt:
(a) Inkubieren der Testeinrichtung nach Anspruch 1 oder 6 mit einer Blutprobe, deren Typus bestimmt werden soll;
(b) Spülen der Testeinrichtung;
(c) Feststellen eines Farbsignals aus Erythrozyten, welche immunologisch an Antikörper gebunden

EP 0 223 978 B1

sind, welche letztgenannten an das poröse, feste Substrat gebunden sind.

**9.** Testeinrichtung zum Bestimmen des Bluttypus einer Blutprobe, welche Testeinrichtung umfaßt:
(a) ein festes Substrat;
(b) eine Blutgruppensubstanz, welche Antigendeterminanten zur Verfügung stellt, welche kennzeichnend für einen spezifischen Bluttypus sind, wobei diese Blutgruppensubstanz derart an das feste Substrat gebunden ist, daß sie befähigt ist, Serum-Antikörper aus einer Serumprobe zu binden, welche Antikörper selbst befähigt sind, solche Erythrozyten immunologisch zu binden, mit welchen die Antikörper spezifisch reaktiv sind, dadurch gekennzeichnet, daß das feste Substrat porös ist, nämlich eine mittlere Porengröße von 0,025 bis 7,0 $\mu$m hat, und daß die Blutgruppensubstanz weiterhin Blutgruppensubstanzen von mehr als einem spezifischen Bluttypus umfassen kann, wobei jede Blutgruppensubstanz eines spezifischen Bluttypus an das feste Substrat in einer abgegrenzten Adsorptionsfläche gebunden ist, um innerhalb der abgegrenzten Fläche - im Gegensatz zu Flächenteilen auf der Oberfläche des Substrates, auf welchen keine Blutgruppensubstanzen gebunden sind - ein feststellbares Farbsignal zu erzeugen.

**10.** Testeinrichtung nach Anspruch 9, wobei das poröse, feste Substrat ein Nitrozelluloseblatt ist.

**11.** Testeinrichtung nach Anspruch 9, wobei die Blutgruppensubstanz aus einer Gruppe ausgewählt ist, welche aus Blutgruppensubstanzen besteht, welche eine Antigendeterminante zur Verfügung stellen, die für die Bluttypen A oder B kennzeichnend ist.

**12.** Testeinrichtung nach Anspruch 9, wobei die Blutgruppensubstanz ein Oligosaccharid ist.

**13.** Testeinrichtung nach Anspruch 9, wobei die Blutgruppensubstanz eine lysierte Erythrozytenmembran mit einer charakteristischen Bluttypenspezifität ist.

**14.** Verfahren zum Bestimmen des Bluttypus einer Blutprobe, welches Verfahren umfaßt:
(a) Inkubieren der Testeinrichtung nach Anspruch 9 mit einer Probe von Vollblut oder von aus Vollblut isoliertem Serum, dessen Typus zu bestimmen ist;
(b) Spülen der Testeinrichtung;
(c) Inberührungbringen der Testeinrichtung mit einem Gemisch von Erythrozyten, welches Erythrozyten umfaßt, welche Antigendeterminanten darbieten, welche kennzeichnend für die Bluttypen sind, auf welche untersucht werden soll;
(d) Feststellen eines Farbsignals aus Erythrozyten, welche über die Serum-Antikörper an die Blutgruppensubstanz gebunden sind, welche letztgenannte an das feste Substrat gebunden ist.

**15.** Verfahren nach Anspruch 14, wobei das Gemisch von Erythrozyten solche vom Typus A und solche vom Typus B umfaßt.

**16.** Verfahren nach Anspruch 14, wobei das Gemisch von Erythrozyten solche des Typus AB umfaßt.

**Revendications**

**1.** Dispositif de test pour déterminer le type sanguin d'un échantillon de sang, comprenant :
(a) un substrat solide ;
(b) un ou plusieurs anticorps sensiblements purs, spécifiquement réactifs avec les déterminants antigéniques érythrocytaires caractéristiques d'un type sanguin, chaque anticorps étant lié audit substrat dans une zone d'adsorption délimitée et étant capable de se lier immunologiquement à la surface desdits érythrocytes de l'échantillon de sang pour lesquels l'anticorps est spécifiquement réactif, de manière à produire un signal coloré détectable dans la zone délimitée par contraste avec les zones de la surface dudit substrat dans lesquelles aucun anticorps de type sanguin n'est lié, caractérisé en ce que ledit substrat solide est poreux et a une taille moyenne de pores de 0,025 à 7,0 $\mu$m.

**2.** Dispositif de test selon la revendication 1, dans lequel ledit substrat est une feuille de nitrocellulose.

**3.** Dipositif de test selon la revendication 1, dans lequel les anticorps sont liés audit substrat de manière

14

non covalente.

4. Dipositif de test selon la revendication 1, dans lequel les anticorps sont des anticorps purifiés par affinité.

5. Dispositif de test selon la revendication 1, dans lequel les anticorps sont choisis dans le groupe formé par les anticorps anti-A, anti-B et anti-D.

6. Dispositif de test selon la revendication 2, dans lequel les anticorps sont un ou plusieurs anticorps sensiblements purs choisis dans le groupe formé par les anticorps monoclonaux anti-A purifiés par affinité, les anticorps monoclonaux anti-B purifiés par affinité et les anticorps polyclonaux anti-D purifiés par affinité, chaque anticorps étant lié à la feuille de manière non covalente.

7. Dispositif de test selon la revendication 6, dans lequel la feuille de nitrocellulose a une taille de pores d'environ 0,45 $\mu$m.

8. Procédé pour déterminer le type sanguin d'un échantillon de sang, comprenant :
(a) l'incubation du dispositif de test de la revendication 1 ou 6 avec un échantillon de sang dont le type doit être déterminé ;
(b) le rinçage du dispositif de test ;
(c) la détection d'un signal coloré provenant des érythrocytes liés immunologiquement aux anticorps liés au substrat solide poreux.

9. Dispositif de test pour déterminer le type sanguin d'un échantillon de sang, comprenant :
(a) un substrat solide ;
(b) une substance de groupe sanguin fournissant des déterminants antigéniques caractéristiques d'un type sanguin spécifique, cette substance de groupe sanguin étant liée au substrat solide de telle manière qu'elle est capable de se lier aux anticorps sériques provenant d'un échantillon de sérum, ces anticorps étant eux-mêmes capables de se lier immunologiquement aux érythrocytes avec lesquels les anticorps sont spécifiquement réactifs, caractérisé en ce que ledit substrat solide est poreux et a une taille moyenne de pores de 0,025 à 7,0 $\mu$m et en ce que ladite substance du groupe sanguin peut être en outre des substances du groupe sanguin de plus d'un type sanguin spécifique, chaque substance de groupe sanguin d'un type sanguin spécifique étant liée au substrat solide dans une zone d'adsorption délimitée de manière à produire un signal coloré détectable dans la zone délimitée par contraste avec les zones de la surface dudit substrat dans lesquelles aucune substance du groupe sanguin n'est liée.

10. Dipositif de test selon la revendication 9, dans lequel le substrat solide poreux est une feuille de nitrocellulose.

11. Dispositif de test selon la revendication 9, dans lequel la substance de groupe sanguin est choisie dans le groupe formé par les substances de groupe sanguin fournissant un déterminant antigénique caractéristique des types sanguins A ou B.

12. Dispositif de test selon la revendicaton 9, dans lequel la substance de groupe sanguin est un oligosaccharide.

13. Dispositif de test selon la revendication 9, dans lequel la substance de groupe sanguin est une membrane d'érythrocyte lysé d'une spécificité de type sanguin caractéristique.

14. Procédé pour déterminer le type sanguin d'un échantillon de sang, comprenant :
(a) l'incubation du dispositif de test de la revendication 9 avec un échantillon de sang total ou de sérum isolé de sans total dont le type doit être déterminé ;
(b) le rinçage du dispositif de test ;
(c) la mise en contact du dispositif de test avec un mélange d'érythrocytes comprenant des érythrocytes présentant des déterminants antigéniques caractéristiques des types sanguins à tester ;
(d) la détection d'un signal coloré provenant des érythrocytes liés par l'intermédiaire des anticorps sériques à la substance du groupe sanguin liée au substrat solide.

15

**15.** Procédé selon la revendication 14, dans lequel le mélange d'érythrocytes comprend des érythrocytes de type A et des érythrocytes de type B.

**16.** Procédé selon la revendication 14, dans lequel le mélange d'érythrocytes comprend des érythrocytes de type AB.